# EUROPEAN PATENT APPLICATION

(11) **EP 3 485 725 A1**
(43) Date of publication of application: **22.05.2019**
(21) Application number: 16906171.0
(22) Date of filing: 21.12.2016
(51) Int. Cl.: A01H 1/02, A01H 1/04, A01H 1/08, A01H 4/00

(54) **METHOD FOR BREEDING CRUCIFER VEGETABLE MATERIAL AND VARIETIES BY DOUBLE HAPLOID INDUCING LINE OF RAPE**

(30) Priority: 23.06.2016 CN 201610458209
(71) Applicant: Chengdu Academy of Agriculture and Forestry Sciences, Chengdu, Sichuan 611130 (CN)
(72) Inventor: YANG, Jin, Chengdu Sichuan 611130 (CN); FU, Shaohong, Chengdu Sichuan 611130 (CN); KUANG, Chengbing, Chengdu Sichuan 611130 (CN); CHEN, Ling, Chengdu Sichuan 611130 (CN); LI, Yun, Chengdu Sichuan 611130 (CN); TANG, Zujun, Chengdu Sichuan 611130 (CN); WANG, Jisheng, Chengdu Sichuan 611130 (CN); ZOU, Qiong, Chengdu Sichuan 611130 (CN); TAO, Lanrong, Chengdu Sichuan 611130 (CN); KANG, Zeming, Chengdu Sichuan 611130 (CN); TANG, Rong, Chengdu Sichuan 611130 (CN)
(74) Representative: Roman, Alexis
(86) International application number: PCT/CN2016/111356
(87) International publication number: WO 2017/219634

(57) **Abstract**

The present invention discloses a method for breeding cruciferous vegetable materials and varieties with a double haploid induction line of rapeseed, including: 1) collecting resources of cruciferous vegetables, identifying and classifying same, and regulating the sowing time of the double haploid induction line of rapeseed to ensure flower synchronization; 2) pollinating the cruciferous vegetables with the double haploid induction line of rapeseed; 3) performing bagged selfing or bud peeling forced selfing at a bud stage on the induced progeny individual plants; 4) identifying the strain stability of the induced selfing progenies; 5) test-crossing stable strains with sterile lines; 6) investigating the sterile degree of test-cross progenies to breed excellent maintainer lines; 7) pollinating sterile plants of the test-cross progenies with the double haploid induction line of rapeseed; 8) investigating the fertility of the induced progenies, and continuing to pollinate sterile individual plants with the inducing line to breed new sterile lines; and 9) test-matching the sterile lines with the maintainer lines to breed new hybrid combinations or new hybrid varieties. The method of the present invention can be flexibly applied in cruciferous vegetables, and can greatly improve the breeding efficiency of cruciferous vegetables and reduce the cost of manpower and material resources.

## Description

### BACKGROUND

### Technical Field

The present invention relates to agriculture, and in particular to a method for breeding new hybrid varieties of cruciferous vegetables and quickly breeding sterile lines and maintainer lines.

### Related Art

Cruciferous vegetables are a large class of winter vegetables in China, also a main source of winter vegetables in China, mainly including *Brassica oleracea,* cauliflower (broccoli), Chinese cabbage (celery cabbage, pakchoi), radish, mustard cruciferous vegetables (green vegetables, mustard, kohlrabi, Chinese kale, etc.). Breeding of hybrid varieties of cruciferous vegetables have been substantially achieved at present. The main heterosis way is mainly based on cytoplasmic male sterile types. The hybrid varieties of *Brassica oleracea,* cauliflower (broccoli) and radish are bred mainly based on radish cytoplasmic male sterile types, and the hybrid varieties of other cruciferous vegetables are bred mainly based on cytoplasmic male sterile types. Since the main purpose of cruciferous vegetables is to obtain vegetative bodies, the breeding of varieties is mainly for the purpose of breeding sterile lines and maintainer lines, and no restorer lines are needed. Good hybrid combinations or new varieties can be bred by test matching of new maintainer lines and sterile lines.

For breeding a new variety of cruciferous vegetables, a new inbred line or genetically stable homozygous strains-homozygous line (inbred line) is bred first. Second, the homozygous strains are test-crossed with a cytoplasmic male sterile line to judge a restoring and maintaining relationship. If a maintainer line is crossed with a sterile line to test-match a new hybrid variety, or the maintainer line is back-crossed by multiple generations to breed a sterile line having the characteristics of the maintainer line, a batch of good combinations or varieties can be bred by test-matching the new sterile line with multiple maintainer lines. If the homozygous strains are test-crossed with the sterile line and the test-cross progenies are not restored or maintained, the homozygous strains are generally eliminated, or enter next round of breeding of maintainer lines. In order to realize hybrid popularization of cruciferous vegetables, the homozygous inbred lines currently selected have the restoring efficiency and can still be used for variety breeding. However, for resource protection, breeders tend to breed maintainer lines.

The breeding of conventional inbred lines of cruciferous vegetables is realized in such a manner that two or more lines with different genetic backgrounds are crossed, convergently crossed or back-crossed to form a hybrid F₁ generation (or a back-cross generation, and multi-generation back crossing can be performed according to the selection requirements of target traits to form BC2, BC3, ...), the back-cross posterity or the F₁ generation is selfed to form an F₂ generation, excellent individual plants are selected from the F₂ generation and selfed to form an F₃ generation, individual plants are selected from F₃ and selfed, and new stable lines of cruciferous vegetables can be obtained till F₅ to F₆, which takes about 6-7 years, calculated by one generation per year. The stable inbred lines are then test-matched with sterile lines, and new sterile lines are bred by 5 to 6 generations of back-crossing. Therefore, the two-line breeding of new varieties of cruciferous vegetables by conventional means takes about 10-12 years, so that the efficiency of breeding new hybrid combinations or varieties is very low.

At present, inducing lines or double haploid inducing lines have not been reported in cruciferous vegetables. The "inducing lines" indicate that the pollen of a kind of plants as male parents is used to pollinate the same kind of plants, and the same kind of plants (female parents) can be induced to produce the corresponding effects, e.g., produce haploids, double haploids (DH lines), etc. Maize is mostly used among plants for breeding new varieties by inducing lines, but the inducing lines in maize are only haploid inducing lines. The earliest maize haploid inducing line was stock6, which can induce maize to produce only haploids, and then the haploid plants were doubled by artificial chromosomes to form homozygous diploids (double haploids), and the inducing efficiency is low, generally 10% or less (calculated by the number of haploids obtained from harvested seeds).

### SUMMARY

The object of the present invention is to provide a method capable of quickly and effectively breeding cruciferous vegetable materials and varieties. The method requires only 3 generations (2 years or 3 years) to obtain genetically stable strains of cruciferous vegetables, thereby improving the efficiency and pertinence of breeding materials and hybrid varieties of cruciferous vegetables.

The object of-the present invention is achieved in this way:
A method for breeding cruciferous vegetable materials and varieties with a double haploid induction line of rapeseed according to the present invention comprises the following steps:
1) collecting breeding resources of cruciferous vegetables and identifying the characteristics of resource materials, including disease resistance, stress resistance, agronomic traits, yield, quality, etc., and classifying and numbering the resource materials having different sources and a great genetic difference of agronomic traits; investigating the flowering time, and retarding to sow the double haploid induction line of rapeseed according to the flowering time, the sowing time of rapeseed double haploids being generally October 20 to November 5 of the previous year, which can ensure flower synchronization with cruciferous vegetables such as *Brassica oleracea,* Chinese cabbage, radish and cauliflower in the next year;
2) artificially castrating the resource materials identified to be good but genetically unstable in step 1) at the initial flowering stage (sterile resources can be directly pollinated), performing bagging isolation for 2-4 days, then performing pollination with the pollen of double haploid induction line of rapeseed, and harvesting induced progenies from the bagged individual plants;
3) planting the induced progenies harvested in step 2), identifying the ploidies of the induced progenies with a flow cytometer at the seedling stage to eliminate polyploids, haploids and plants with rapeseed characteristics, and selecting individual plants normal in fertility and ploidy for bagged selfing or bud peeling forced selfing at the bud stage;
4) planting strains of normal selfing progenies of the induced progenies in step 3), identifying the stability and consistency in the strains, identifying the consistency in the strains with molecular markers (SSR or SRAP), test-crossing the stable strains as male parents with a stable cytoplasmic male sterile line (cytoplasmic male sterile type such as radish cytoplasmic male sterile type) as female parents, and harvesting test-cross progeny seeds;
5) planting the test-cross seeds in step 4), and identifying the fertility of the test-cross progenies, wherein if the test-cross progenies are completely sterile, the corresponding male parents in step 4) are a maintainer line of the sterile type, and meanwhile the selfing setting rate of the maintainer line is identified; wherein if the test-cross progenies are completely fertile, the corresponding male parents in step 4) are a restorer line of the sterile type, and the corresponding restorer line is eliminated; wherein if the test-cross progenies are sterile incompletely (half restored and half maintained), the corresponding male parents in step 4) are not restored or maintained, and are eliminated or crossed to breed new maintainer lines;
6) test-matching the maintainer line bred in step 5) with the same type of sterile line to breed a new hybrid combination or variety with the characteristics of yield, quality and disease resistance;
7) test-crossing the maintainer line bred in step 5) with the same type of sterile line, and then according to the agronomic traits of the test-cross progenies, performing back crossing or multi-generation back crossing on the maintainer line with the test-cross progenies, or pollinating the sterile individual plants of test crossing, back crossing or multi-generation back crossing with the double haploid induction line of rapeseed at the test-cross first generation, and isolating same by bagging;
8) planting individual plant seeds harvested in step 7), identifying the fertility at the flowering stage, continuing to pollinate and induce sterile individual plants with the double haploid induction line of rapeseed, bagging the individual plants for isolation, and harvesting seeds from the individual plants;
9) identifying the in-strain stability and consistency of the second induced and sterile individual plant progenies in step 8) through agronomic traits and molecular markers (SSR or SRAP); forming a new cytoplasmic male sterile line of cruciferous vegetables for in-strain stable sterile strains having consistent agronomic traits, and performing third induced pollination on the sterile strains with the double haploid induction line of rapeseed to identify the inducing efficiency;
10) identifying the inducing capability of the inducing line to the sterile strains for the progenies of third induction in step 9), and maintaining the genetic characteristics and infertility of the newly formed sterile strains (or stable sterile line) using the double haploid induction line of rapeseed if the agronomic traits and the infertility in the third induced progeny strains are highly consistent, and the inducing capability exceeds 98% (a ratio of plants with highly consistent agronomic traits and infertility to the total induced progenies);
11) pollinating the sterile strains formed after second induction in step 8) with the double haploid induction line of rapeseed to maintain the infertility thereof, the genetic characteristics of the sterile strains (or stable sterile line) being irrelevant to the double haploid induction line of rapeseed, the sterile strains having the traits of the maintainer line (temporary maintainer line) in step 7), and having a genetic difference from the maintainer line (temporary maintainer line), or containing 50-99% of nuclear genes of the maintainer line (temporary maintainer line), the content of the nuclear genes of the maintainer line (temporary maintainer line) depending on the number of back-crossing generations of the maintainer line (temporary maintainer line) and the sterile individual plants, and the sterile strains also containing nuclear genes of the stable cytoplasmic male sterile line in step 4) besides the nuclear genes of different degrees of the maintainer line (temporary maintainer line);
12) maintaining the cytoplasmic male sterile lines (stable sterile line) with genetic differences (or different genetic backgrounds) formed in step 9) with the same maintainer line (double haploid induction line of rapeseed) according to the inducing capability (more than 98%) of the double haploid induction line of rapeseed to the cytoplasmic male sterile line, the double haploid induction line of rapeseed becoming a universal maintainer line of cytoplasmic male sterile lines; meanwhile, maintaining a plurality of genetically stable cytoplasmic male sterile lines with different genetic backgrounds with the same maintainer line;
13) breeding new cruciferous vegetable varieties with yield potential, disease resistance and stress resistance to realize two-line matching breeding of new hybrid varieties of cruciferous vegetables according to the material characteristics having different sources and a great genetic difference of agronomic traits in step 1) and the test matching of the corresponding source-induced stable maintainer line and the new stable sterile line formed in step 12).

Stable genetic progenies of cruciferous vegetables are obtained by the method according to the present invention, wherein the double haploid induction line of rapeseed can induce parthenogenesis of female plants at the F₁ generation, stable double haploid individual plants are formed at the F₂ generation, the stability and the consistency are identified at the F₃ generation, and the stable genetic progenies are thus obtained.

A method for breeding the above-mentioned double haploid induction line of rapeseed comprises the following steps:
(1) breeding an early generation stable line with the parthenogenesis genetic characteristic:
   a. artificially doubling chromosomes of hybrid F₁ generation seeds of two rapeseed parent materials on a medium by using a chromosome doubling inducer to obtain doubled F₁ generation plants;
   b. selfing or forcedly selfing the doubled F₁ generation plants to obtain an F₂ generation, performing field planting observation on the F₂ generation, identifying the fertility of each individual plant, selecting fertile progenies and selfing same to obtain an F₃ generation, identifying the homozygosity of the F₃ generation by morphology, cytology and molecular markers, performing polymerase chain reaction amplification on progeny DNA, and observing the type and number of DNA bands of the individual plants under the amplification of each specific primer by electrophoresis, which shows that each individual plant is a hybrid progeny of two parents, and the molecular marker maps of the individual plants are consistent, indicating that these individual plants are of a homozygous line, i.e. an early generation stable line;
   c. reciprocally crossing the obtained early generation stable line with at least 10 conventional homozygous stable lines of rapeseed, and identifying the genetic characteristics of the early generation stable line at the F₁ and F₂ generations, i.e., identifying whether there is the parthenogenesis characteristic, wherein if F₁ is separated and part of stable strains appear in the F₂ generation in the reciprocal crossing, the corresponding early generation stable line is an early generation stable line with the parthenogenesis genetic characteristic;
(2) breeding polyploid rapeseed with dominant genetic traits, parthenogenesis genetic characteristic and ploidy genetic stability:
   a. crossing the early generation stable line with the parthenogenesis genetic characteristic with rapeseed with dominant traits (e.g., dominant dwarf, purple leaf, mottled leaf, yellow leaf, high erucic acid, etc.) to obtain hybrid F₁ generation seeds, and artificially doubling chromosomes of the hybrid F₁ seeds on a medium by using a chromosome doubling inducer to obtain doubled F₁ plants with dominant traits;
   b. identifying the chromosome ploidies of the doubled F₁ plants with dominant traits through microscopic observation or a flow cytometer, selecting polyploid plants with dominant traits, and eliminating abnormal doubled plants, aneuploid plants and doubled plants without dominant traits, the polyploid plants with dominant traits being mainly hexaploid or octoploid rapeseed plants with ploidy genetic stability, good setting property, parthenogenesis genetic characteristic and dominant traits (e.g., dominant dwarf, purple leaf, mottled leaf, yellow leaf, high erucic acid, etc.);
(3) identifying the double haploid induction line of rapeseed and measuring the inducing capability:
   a. the dominant traits in the polyploid plants with ploidy genetic stability, parthenogenesis genetic characteristic and dominant traits can be used for removing hybrid plants generated in the test-cross progenies, and if dominant plants or aneuploid plants appear in the test-cross progenies, it indicates that the plants are generated by the polyploid plants and female parents and are removed; and
   b. if the individual plant test-cross progenies are completely sterile but have normal ploidies, i.e. diploid or tetraploid rapeseed, and do not have dominant traits, it indicates that the genes of the corresponding male parents of the test-cross progenies do not enter the test-cross progenies, wherein the dominant polyploid plants are of the double haploid induction line of rapeseed.

The above-mentioned double haploid induction line of rapeseed is bred by artificially doubling chromosomes of hybrid F₁ generation seeds of two rapeseed parent materials, or hybrid F₁ generation seeds obtained by crossing the early generation stable line with the parthenogenesis genetic characteristic with rapeseed with dominant traits, on a medium by using a chromosome doubling inducer, and the specific method is as follows:
1) disinfecting the surfaces of the seeds with 75% alcohol for 25-40 seconds, disinfecting same with 0.1% mercury bichloride for 12-17 minutes, then washing away the mercury bichloride on the surfaces of the seeds with sterile water, sucking the water on the surfaces of the seeds with sterile paper, and then inoculating a first medium with the seeds;
2) allowing the seeds to root and sprout on the first medium under the culture conditions: temperature 23-25°C, daylight illumination 12-16 hours, light intensity 2000-3000 lux, night dark culture 8-12 hours, until the plants grow to 1-2 true leaves, and cutting the plants from the hypocotyls for continuing to grow on a second medium;
3) inserting the cut plants into the second medium to continue the culture, and after lateral buds are differentiated, transferring the lateral buds and the plants to a third medium for rooting culture; and
4) hardening seedlings of the plants at room temperature for 3-7 days after the plants grow thick roots after two weeks of rooting culture, taking the plants out, washing away the medium on the plants with tap water, soaking the plants in a soaking buffer solution for 15-30 minutes, and then transplanting the plants to a greenhouse, the greenhouse having a temperature of 16-25°C and a relative humidity of 60-80%, which can ensure that the survival rate of transplanting is 95% or above;
the first medium consists of the following components:

| | |
|---|---|
| MS medium | 1 L |
| 6-benzyl adenine | 0.5-1.5 mg |
| chromosome doubling inducer | 30-70 mg |
| sucrose | 20-30 g |
| agar | 8-10 g, |

the pH value of the first medium is 5.8-6.0;
the second medium consists of the following components:

| | |
|---|---|
| MS medium | 1 L |
| 6-benzyl adenine | 0.5-1 mg |
| chromosome doubling inducer | 20-40 mg |
| sucrose | 20-30 g |
| agar | 8-10 g, |

the pH value of the second medium is 5.8-6.0;
the third medium consists of the following components:

| | |
|---|---|
| MS medium | 1 L |
| α-naphthaleneacetic acid | 0.03-0.5 mg |
| chromosome doubling inducer | 5-20 mg |
| sucrose | 20-30 g |
| agar | 8-10 g, |

the pH value of the third medium is 5.8-6.0;
the soaking buffer solution consists of the following components:

| | |
|---|---|
| water | 1 L |
| famoxadone or curzate | 0.6-1.2 g |
| α-naphthaleneacetic acid | 0.5-1 mg. |

The double haploid induction line of rapeseed can directly induce rapeseed and cruciferous vegetables to produce double haploid progenies without artificial chromosome doubling for obtaining homozygous lines, and has high inducing efficiency, which is up to 100%, generally 50% or more. The main principle that the double haploid inducing line induces female plants to produce double haploids is that the inducing line can induce megaspore germ cells (egg cells) of female plants to produce a parthenogenetic effect, the chromosomes of the egg cells can be doubled, i.e., the progenies generated by parthenogenesis of the egg cells are double haploids, and the mechanism of such a phenomenon is still unclear.

The above chromosome doubling inducer is at least one of colchicine, trifluralin and oryzalin.

The basic principle of the double haploid induction line of rapeseed (hexaploid or octoploid plants) is that the inducing line has parthenogenetic induction genes; and when the inducing line serves as male parents, the chromosomes (or genes) of the inducing line are not fused with chromosomes of female plants, but induce the female plants (i.e. egg cells, diploids) to produce the parthenogenetic effect, and the chromosomes of the egg cells of the female plants are doubled to form double haploids.

The method of the present invention can be used for rapidly breeding inbred lines (DH lines), maintainer lines and new cytoplasmic male sterile lines of cruciferous vegetable breeding materials. The above materials can be obtained within 2 years or 3 generations, so that the breeding time of cruciferous vegetables is greatly saved and the breeding efficiency is improved.

The method of the present invention has the following advantages:
1. The method of the present invention can be used for rapidly breeding inbred lines (maintainer lines) of cruciferous vegetables to obtain stable inherited inbred lines (maintainer lines) fastest within 3 years, and rapidly breeding new sterile lines to obtain stable sterile lines fastest within 4 years, achieve two-line matching and breeding of new varieties of cruciferous vegetables within 5 to 7 years, save half or more of the breeding cycle of cruciferous vegetables, improve the efficiency of breeding new varieties of cruciferous vegetables, and save manpower and material resources.
2. The method of the present invention can be applied to the entire cruciferous vegetables, so that the application field is wide.
3. The double haploid induction line of rapeseed directly induces female plants to produce double haploids without artificial chromosome doubling, and the double haploids can further form stable progenies in one step.
4. The method of the present invention is suitable for breeding of hybrid varieties of cruciferous vegetables, especially for breeding of cytoplasmic male sterile materials of cruciferous vegetables, e.g., breeding of radish cytoplasmic male sterile lines, and cytoplasmic male sterile lines and maintainer lines of nucleo-cytoplasmic interaction types.

### BRIEF DESCRIPTION OF THE DRAWINGS

FIG. 1 is a flowchart of a method for rapidly breeding new varieties of cruciferous vegetables with a double haploid induction line of rapeseed.
FIG. 2 is a flowchart of breeding of a double haploid induction line of rapeseed.
FIG. 3 is a flowchart of a method for obtaining an early generation stable line of rapeseed.
FIG. 4 is a flowchart of breeding of a double haploid induction line of rapeseed Y3560.
FIG. 5 is a flowchart of breeding of a double haploid induction line of rapeseed Y3380.
FIG. 6 is a flowchart of breeding of an early generation stable line P3-2 of rapeseed.
FIG. 7 is a breeding diagram of a *Brassica oleracea* maintainer line Ronggan B012.
FIG. 8 is a breeding diagram of radish cytoplasmic male sterile line Ronggan A105 of *Brassica oleracea.*
FIG. 9 is a breeding diagram of a radish sterile line Luorong A007.
FIG. 10 is a diagram of identifying ploidies of chromosomes at root tips of P3-2 tetraploid rapeseed.
FIG. 11 is a diagram of identifying ploidies of French cells of P3-2 tetraploid rapeseed.
FIG. 12 is a flow cytometry ploidy identification diagram of Y3380.
FIG. 13 is a flow cytometry ploidy identification diagram of Y3560.

### DETAILED DESCRIPTION

### Embodiment 1:

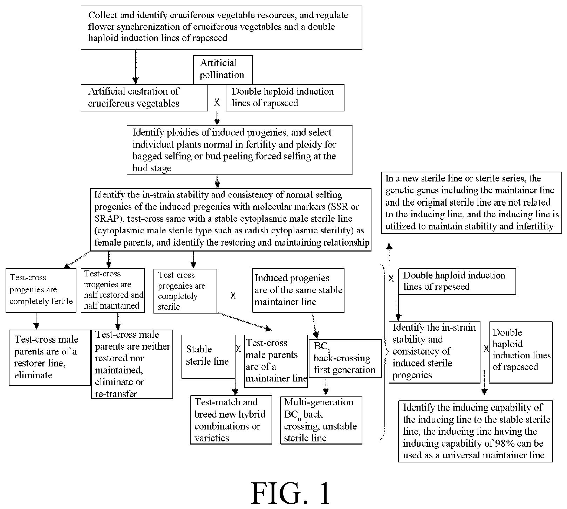

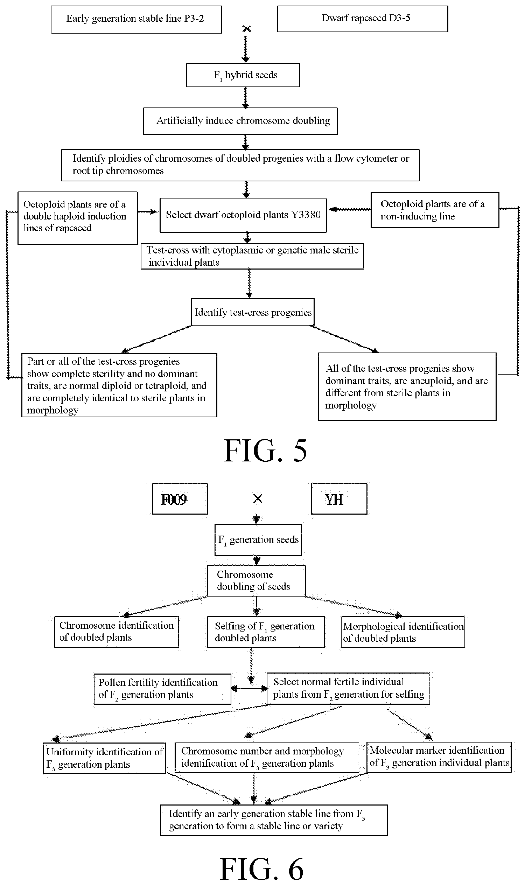

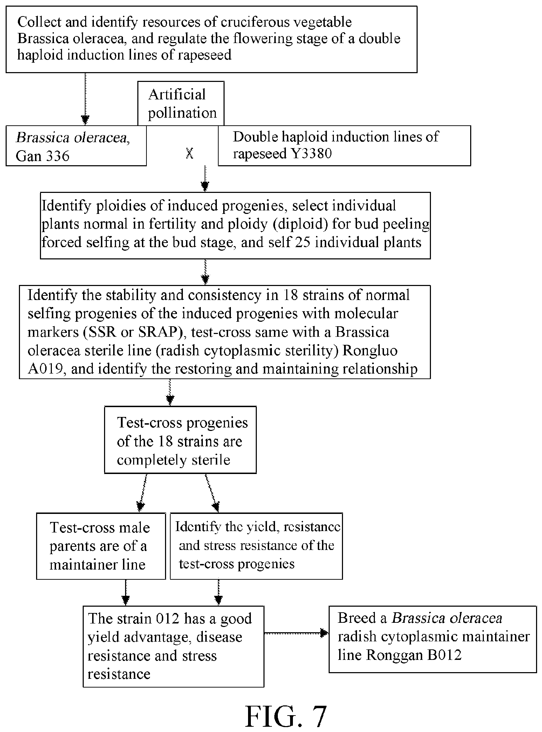

Referring to FIG. 1, FIG. 2, FIG. 5 and FIG. 7, *Brassica oleracea* resources collected for many years were planted in field, and the traits were observed. It was discovered that Gan 336 had a good appearance, high yield and disease resistance, but was unstable in groups with genetic separation. Seeds were sowed in mid-August of last year, seedlings were planted in mid-September and replanted in late December while vegetative growth was removed, Gan 336 was artificially castrated during the early flowering stage at the end of March of the first year and bagged for isolation, and the Gan 336 was induced by a double haploid induction line of rapeseed Y3380 after being castrated for 3 days, pollinated and bagged for isolation. The induced progenies were sowed in field in August of the same year, and 25 progenies were discovered to be completely identical to *Brassica oleracea* in appearance and identified to be all diploids with a flow cytometer. The progeny individual plants were subjected to bud peeling forced selfing at the flowering stage of the second year and bagged for isolation, and 18 strains were harvested. The induced progeny strains were identified in the third year, and all the 18 strains were found to be consistent with some differences. The 18 strains were test-crossed with a *Brassica oleracea* sterile line (radish cytoplasmic male sterile line) Rongluo A019, the test-cross progenies of the 18 stable strains were highly sterile, and the 18 stable strains were maintainer lines for the sterile line. It was discovered by morphological observation and yield investigation that the 012 strain had better yield, disease resistance and stress resistance. A maintainer line of Ronggan B012 was finally formed, and could be directly combined with the radish cytoplasmic male sterile line of *Brassica oleracea* to breed new varieties.

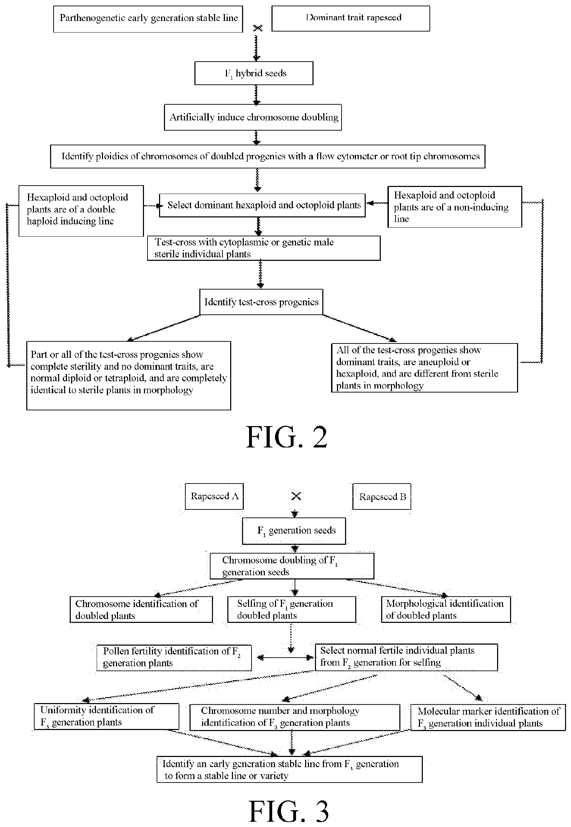

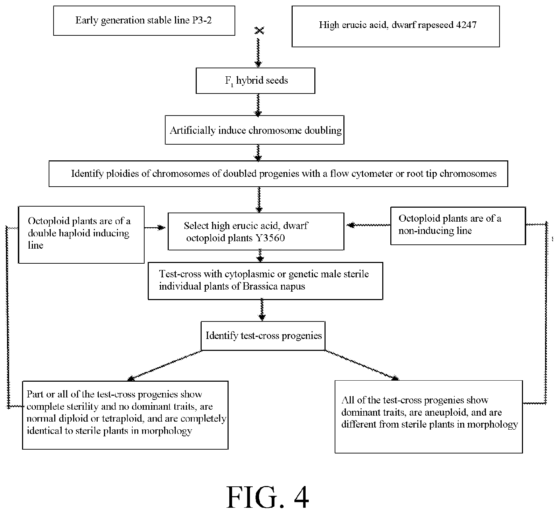

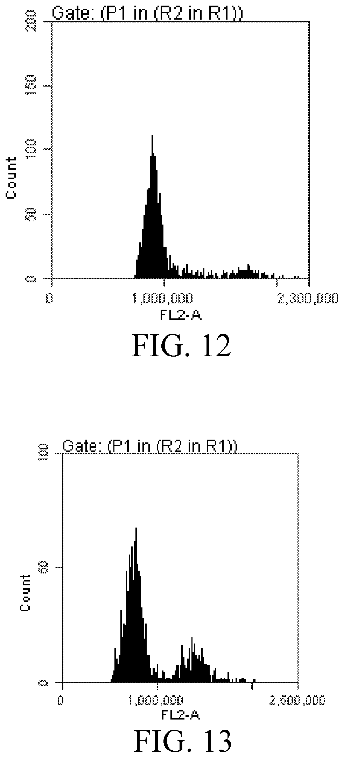

In this embodiment, the double haploid induction line of rapeseed was obtained by the following method:
Referring to FIG. 2, FIG. 4, FIG. 6, FIG. 7 and FIG. 13, the tetraploid early generation stable line P3-2 of *Brassica napus* obtained by the applicant was reciprocally crossed with 20 homozygous tetraploid *Brassica napus,* three reciprocal cross F₁ generations were isolated, and the combined F₂ generation of the three showed stable strains, indicating that P3-2 has the parthenogenesis genetic characteristic. P3-2 was reciprocally crossed with high erucic acid, dwarf rapeseed 4247 (dwarf and high erucic acid were dominant traits), then hybrid F₁ generation seeds were subjected to chromosome doubling, and doubled progenies were identified by a flow cytometer or root tip microscopic observation to show dwarf octaploid plants named Y3560.

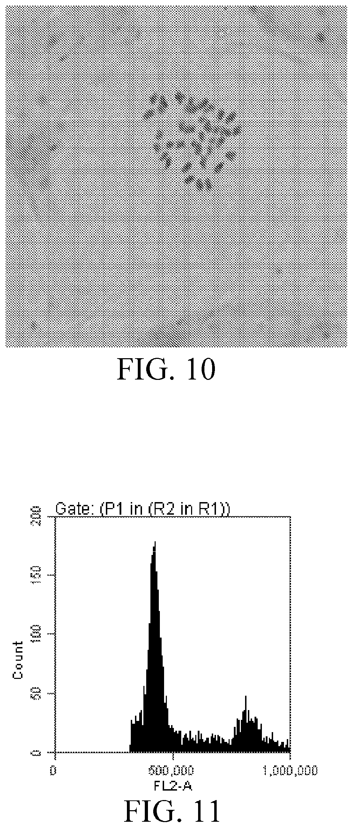

Referring to FIG. 2, FIG. 5, FIG. 6, FIG. 10, FIG. 11 and FIG. 12, the tetraploid early generation stable line P3-2 of *Brassica napus* obtained by the applicant was reciprocally crossed with 20 homozygous tetraploid *Brassica napus,* three reciprocal cross F₁ generations were isolated, and the combined F₂ generation of the three showed stable strains, indicating that P3-2 has the parthenogenesis genetic characteristic. P3-2 was reciprocally crossed with tetraploid dwarf *Brassica napus* D3-5 (dwarf was a dominant trait), then hybrid F₁ generation seeds were subjected to chromosome doubling, and doubled progenies were identified by a flow cytometer or root tip microscopic observation to show dwarf octaploid plants named Y3380.

In this embodiment, the specific method of artificial chromosome doubling for hybrid F₁ seeds of P3-2 and dwarf *Brassica napus* D3-5, as well as hybrid F₁ seeds of P3-2 and dwarf, high erucic acid rapeseed 4247 on a medium with colchicine was as follows:
1) disinfecting the surfaces of the seeds with 75% alcohol for 25 seconds, disinfecting same with 0.1% mercury bichloride for 12 minutes, then washing away the mercury bichloride on the surfaces of the seeds with sterile water, sucking the water on the surfaces of the seeds with sterile paper, and then inoculating the seeds onto a first medium (chromosome doubling inducing medium);
2) allowing the seeds to root and sprout on the first medium under the culture conditions: temperature 25°C, daylight illumination 16 hours, light intensity 2000 lux, night dark culture 8 hours, until the plants grew to 1-2 true leaves, and cutting the plants from the hypocotyls for continuing to grow on a second medium;
3) inserting the cut plants into the second medium to continue the culture, and after lateral buds were differentiated, transferring the lateral buds and the plants to a third medium (rooting medium) for rooting culture; and
4) hardening seedlings of the plants at room temperature for 3 days after the plants grew thick roots after two weeks of rooting culture, taking the plants out, washing away the medium on the plants with tap water, soaking the plants in a soaking buffer solution for 15 minutes, and then transplanting the plants to a greenhouse, the greenhouse having a temperature of 25°C and a relative humidity of 60%, which can ensure that the survival rate of transplanting was 95% or above;
the first medium consisted of the following components:

| | |
|---|---|
| MS medium | 1 L |
| 6-benzyl adenine (6BA) | 0.5 mg |
| colchicine | 50 mg |
| sucrose | 20 g |
| agar | 8 g, |

the pH value of the first medium was 5.8-6.0;
the MS medium was invented by Murashige and Skoog, abbreviated as MS, and its formulation was shown in annexed Table 1.
the second medium consisted of the following components:

| | |
|---|---|
| MS medium | 1 L |
| 6-benzyl adenine (6BA) | 0.5 mg |
| colchicine | 30 mg |
| sucrose | 30 g |
| agar | 8 g, |

the pH value of the second medium was 5.8-6.0;
the third medium consisted of the following components:

| | |
|---|---|
| MS medium | 1 L |
| α-naphthaleneacetic acid | 0.03 mg |
| colchicine | 20 mg |
| sucrose | 20 g |
| agar | 8 g, |

the pH value of the third medium was 5.8-6.0;
the soaking buffer solution consisted of the following components:

| | |
|---|---|
| water | 1 L |
| famoxadone or curzate | 0.6 g |
| α-naphthaleneacetic acid | 0.5 mg. |

Referring to FIG. 2, FIG. 3 and FIG. 5, Y3380 as male parents was test-crossed with a cytoplasmic male sterile line (0464A) of *Brassica napus* to obtain 50 test-cross progenies, all of which had high stalks and were tetraploid *Brassica napus,* where 49 strains were completely sterile, 1 strain was semi-sterile, and the morphological characteristics were completely identical to the 0464A. At the same time, hybrid F₁ (non-doubled strains) of P3-2 and dwarf *Brassica napus* D3-5 was used as male parents and test-crossed with 0464A as a contrast for verification to obtain 102 test-cross progenies, which included 62 dwarf plants and 40 high-stalk plants, and were high in fertility separation with 73 completely fertile, 20 semi-sterile and 9 completely sterile. It indicated that the genes in Y3380 did not enter the test-cross plants, the test-cross progenies were parthenogenetic for the 4646A, and the induction rate was 98%. Y3380 as male parents was convergently crossed with castrated *Brassica napus* 3954 (3954 was F₁, obtained by crossing Zhongshuang 11 with CAX), the convergently crossed progenies F₁ were separated, each F₁ was selfed, and 45 F₁ selfed strains were harvested. 45 F₂ generation strains were planted, and 45 stable strains appeared, so that the stable strains showed 100% and the induction rate was 100%.

Y3380 as male parents was convergently crossed with castrated *Brassica napus* 3968 (3968 was F₁, obtained by crossing Zhongshuang 11 with 1365), the convergently crossed progenies F₁ were separated, each F₁ was selfed, and 52 F₁ selfed strains were harvested. 52 F₂ generation strains were planted, and 28 stable strains appeared, so that the stable strains showed 53.85% and the induction rate was 53.85%.

Y3380 as male parents was crossed with castrated *Brassica napus* Zhongshuang 11 (conventional variety, homozygous) to obtain 70 hybrid F₁ plants, the 70 F₁ plants were completely identical to Zhongshuang 11 in shape, and the F₂ generation did not separate after each individual plant was selfed, and showed stable strains that were completely identical to Zhongshuang 11 in shape, indicating that the F₁ generation was homozygous. That is, the crossing process of Y3380 and Zhongshuang 11 induced parthenogenesis in Zhongshuang 11, and the F₁ produced was of parthenogenetic selfing and was homozygous, so that F₁ was stable, F₂ was also stable, F₁ and F₂ were completely identical to Zhongshuang 11 in shape, and the induction rate was 100%.

Similarly, Y3380 as male parents was crossed with castrated *Brassica campestris* Ya'an yellow rapeseed YH (diploid rapeseed, 2n=20) to obtain 98 hybrid F₁ plants, in which 97 F₁ plants were completely identical to YH in shape, and the F₂ generation after each individual plant was selfed was diploid and identical to YH in shape, indicating that the crossing process of Y3380 and YH induced parthenogenesis in YH, the F₁ produced was of parthenogenetic selfing and completely identical to YH in shape, and the induction rate was 98.9%. Finally, dominant dwarf octaploid plants Y3380 were identified as a double haploid induction line of rapeseed.

Referring to FIG. 2, FIG. 3 and FIG. 4, Y3560 as male parents was test-crossed with a cytoplasmic male sterile line (0464A) of *Brassica napus* to obtain 80 test-cross progenies, all of which had high stalks, 76 plants were tetraploid *Brassica napus,* 2 plants were diploid and 2 plants were octaploid, where the 76 tetraploid plants were completely sterile, the 4 plants were semi-sterile, and the morphological characteristics were completely identical to the 0464A. At the same time, hybrid F₁ (non-doubled strains) of P3-2 and dwarf, high erucic acid rapeseed 4247 was used as male parents and test-crossed with 0464A as a contrast for verification to obtain 153 test-cross progenies, which included 102 dwarf plants and 51 high-stalk plants, and were high in fertility separation with 65 completely fertile, 35 semi-sterile and 53 completely sterile. It indicated that the genes in Y3560 did not enter the test-cross plants, the test-cross progenies were parthenogenetic for the 4646A, and the induction rate was 95%.

Y3560 as male parents was crossed with castrated *Brassica campestris* Ya'an yellow rapeseed YH (diploid rapeseed, 2n=20) to obtain 145 hybrid F₁ plants, in which 143 F₁ plants were completely identical to YH in shape, and the F₂ generation after each individual plant was selfed was diploid and identical to YH in shape, indicating that the crossing process of Y3560 and YH induced parthenogenesis in YH, the F₁ produced was of parthenogenetic selfing and completely identical to YH in shape, and the induction rate was 98.6%.

Similarly, Y3560 as male parents was crossed with castrated *Brassica juncea* GW (tetraploid rapeseed, 2n=36) to obtain 124 hybrid F₁ plants, in which 123 F₁ plants were completely identical to GW in shape, and the F₂ generation after each individual plant was selfed was tetraploid and identical to GW in shape, indicating that the crossing process of Y3560 and GW induced parthenogenesis in GW, the F₁ produced was of parthenogenetic selfing and completely identical to GW in shape, and the induction rate was 99.2%. Finally, dominant dwarf octaploid plants Y3560 were identified as a double haploid induction line of rapeseed.

Referring to FIG. 3, FIG. 6, FIG. 10 and FIG. 11, the method for obtaining the early generation stable line P3-2 was as follows:
performing artificial castrated crossing on *Brassica napus* F009 (tetraploid, chromosomes 2n=38) and *Brassica campestris* YH (diploid, Ya'an yellow rapeseed, chromosomes 2n=20) from which buds were peeled to obtain F₁ generation hybrid seeds; performing artificial chromosome doubling on the F₁ generation hybrid seeds with colchicine on a medium; selfing (or forcedly selfing) doubled F₁ generation plants to obtain an F₂ generation, performing field planting observation on the F₂ generation, and identifying the fertility by dyeing pollen with acetic acid magenta to judge the fertility of the pollen, where three cases may occur (1. haploid plants, with little pollen and extremely low fertility; 2. polyploid plants completely sterile, with the development of floral organs impaired, failing to flower normally, having no pollen; 3. normal fertile plants, with more pollen, pollen fertility 95% or more); selfing normal fertile plants of the F₂ generation to obtain an F₃ generation; identifying the homozygosity of the F₃ generation, and planting individual plants of the F₃ generation, where 32% of the individual plants were uniform and normal in flowering and seed setting; performing cytological identification on the uniform plants, showing that the number of chromosomes was consistent (38) and the chromosome morphology was normal; marking with SSR molecular markers, performing DNA polymerase chain reaction, observing the DNA band type of each individual plant by electrophoresis under the amplification of each specific primer, showing that each individual plant was a hybrid progeny of F009 and YH, and the number and type of DNA amplification bands of the individual plants were consistent, and it can be judged that these plants were homozygous, that is, early generation stable lines; and naming one of the early generation stable lines of *Brassica napus* (38 chromosomes) with large leaves, no cleft leaves, compact leave and an oil content of 55% as P3-2.

In this embodiment, the specific method of performing artificial chromosome doubling on the F₁ generation hybrid seeds with colchicine on a medium was as follows:
1) disinfecting the surfaces of the seeds with 75% alcohol for 25 seconds, disinfecting same with 0.1% mercury bichloride for 12 minutes, then washing away the mercury bichloride on the surfaces of the seeds with sterile water, sucking the water on the surfaces of the seeds with sterile paper, and then inoculating the seeds onto a first medium (chromosome doubling inducing medium);
2) allowing the seeds to root and sprout on the first medium under the culture conditions: temperature 25°C, daylight illumination 16 hours, light intensity 2000 lux, night dark culture 8 hours, until the plants grew to 1-2 true leaves, and cutting the plants from the hypocotyls for continuing to grow on a second medium;
3) inserting the cut plants into the second medium to continue the culture, and after lateral buds were differentiated, transferring the lateral buds and the plants to a third medium (rooting medium) for rooting culture; and
4) hardening seedlings of the plants at room temperature for 3 days after the plants grew thick roots after two weeks of rooting culture, taking the plants out, washing away the medium on the plants with tap water, soaking the plants in a soaking buffer solution for 15 minutes, and then transplanting the plants to a greenhouse, the greenhouse having a temperature of 25°C and a relative humidity of 60%, which can ensure that the survival rate of transplanting was above 95%;
the first medium consisted of the following components:

| | |
|---|---|
| MS medium | 1 L |
| 6-benzyl adenine (6BA) | 0.5 mg |
| colchicine | 30 mg |
| sucrose | 20 g |
| agar | 8 g, |

the pH value of the first medium was 5.8-6.0;
the MS medium was invented by Murashige and Skoog, abbreviated as MS, and its formulation was shown in annexed Table 1.
the second medium consisted of the following components:

| | |
|---|---|
| MS medium | 1 L |
| 6-benzyl adenine (6BA) | 0.5 mg |
| colchicine | 20 mg |
| sucrose | 30 g |
| agar | 8 g, |

the pH value of the second medium was 5.8-6.0;
the third medium consisted of the following components:

| | |
|---|---|
| MS medium | 1 L |
| α-naphthaleneacetic acid | 0.03 mg |
| colchicine | 5 mg |
| sucrose | 20 g |
| agar | 8 g, |

the pH value of the third medium was 5.8-6.0;
the soaking buffer solution consisted of the following components:

| | |
|---|---|
| water | 1 L |
| famoxadone or curzate | 0.6 g |
| α-naphthaleneacetic acid | 0.5 mg |

**Table 1 MS medium ingredients**

| Ingredient | Molecular weight | Concentration (mg/L) |
|---|---|---|
| Major element | | |
| Potassium nitrate KNO3 | 101.21 | 1900 |
| Ammonium nitrate NH4NO3 | 80.04 | 1650 |
| Potassium dihydrogen phosphate KH2PO4 | 136.09 | 170 |
| Magnesium sulfate MgSO4•7H2O | 246.47 | 370 |
| Calcium chloride CaC12•2H2O | 147.02 | 440 |

| Trace element | | |
|---|---|---|
| Potassium iodide KI | 166.01 | 0.83 |
| Boric acid H3BO3 | 61.83 | 6.2 |
| Manganese sulfate MnSO4•4H2O | 223.01 | 22.3 |
| Zinc sulfate ZnSO4•7H2O | 287.54 | 8.6 |
| Sodium molybdate Na2MoO4•2H2O | 241.95 | 0.25 |
| Copper sulfate CuSO4•5H2O | 249.68 | 0.25 |
| Cobalt chloride CoC12•6H2O | 237.93 | 0.025 |

| Iron salt | | |
|---|---|---|
| Disodium edetate Na2.EDTA | 372.25 | 37.25 |
| Ferrous sulfate FeSO24•7H2O | 278.03 | 27.85 |

| Organic ingredients | | |
|---|---|---|
| Inositol | | 100 |
| Glycine | | 2 |
| Thiamine hydrochloride VB 1 | | 0.1 |
| Pyridoxine hydrochloride VB6 | | 0.5 |
| Niacin VB5 or VPP | | 0.5 |
| Sucrose | 342.31 | 30 g/L |
| pH | 5.8-6.0 | |

### Embodiment 2:

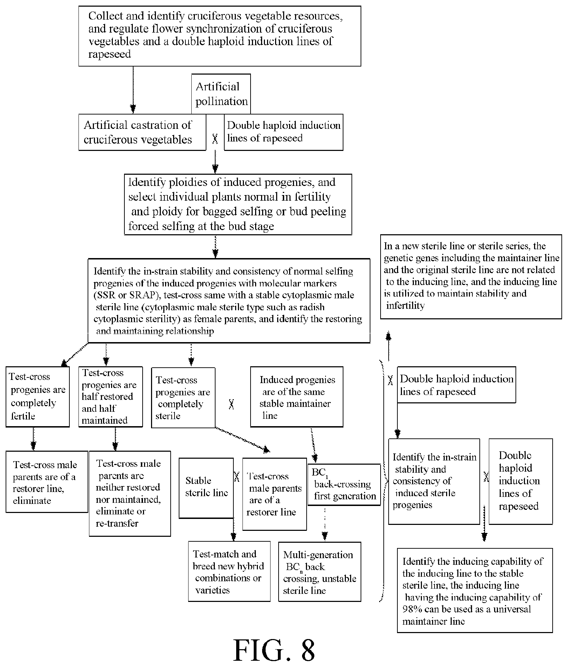

Referring to FIG. 1, FIG. 2, FIG. 4, FIG. 5 and FIG. 8, in order to transform the original *Brassica oleracea* sterile line and improve the combining ability, disease resistance, storage and transportation of the sterile line, a *Brassica oleracea* self-incompatible line (high combining ability line) Gan 121 was crossed with a storage and transportation resistant line Gan 051, the hybrid F₁ generation was artificially castrated at the flowering stage and induced with a double haploid induction line of rapeseed Y3560 by pollination, and 15 individual plants were obtained at an F₂ generation (induced F₁ generation). The survey found that the 15 individual plants were all in the shape of *Brassica oleracea,* and were all diploid. The 15 individual plants were bagged (with buds peeled) and selfed, pollen was taken and test-crossed with a *Brassica oleracea* sterile line (radish cytoplasmic male sterile line) Rongluo A019, the consistency of in-strain stability of the selfed progenies was identified, the consistency and infertility of the test-crossed progenies were identified, and it was discovered that the selfed progenies were all uniform and diploid, the test-crossed progenies were highly consistent and all sterile but have a strain 105 that was resistant to storage and transportation and flat-end, and the test-crossed progenies were high in yield and good in disease resistance. The double haploid induction line of rapeseed Y3380 was continued to be used to pollinate the corresponding test-crossed progeny individual plants of the strain 105, and individual plant seeds were isolated and harvested. The induced progenies of the test-crossed strain 105 were planted in field to obtain 45 plants, in which 42 plants were found to completely have the characteristics of *Brassica oleracea* and were diploid, 3 plants looked like plants crossed with rapeseed and were triploid, and the 42 plants were highly sterile. The sterile individual plants with the characteristics of storage and transportation resistance and flat ends were continued to be pollinated with Y3560 and bagged for isolation, seeds were harvested from the individual plants to obtain 30 strains of plant seeds, and the in-strain consistency and stability of the 30 strains were identified by morphology, agronomic traits and molecular markers (SRAP), where 10 strains were highly uniform in in-strain consistency and stability, and 2 strains had the characteristics of storage and transportation resistance, disease resistance and high yield. 10 plants were selected from each of the 2 strains, 5 plants were pollinated with Y3560 and bagged for isolation, and the other 5 plants were pollinated with Y3380 and bagged for isolation, 5 strains were pollinated in a mixed manner, and the inducing efficiency of the two inducing lines to the stable sterile lines was identified to find that the inducing efficiency of Y3560 to one of the sterile lines was 100% and the inducing efficiency to the other sterile line was 97%, and the inducing efficiency of Y3380 to the two sterile lines was 97% and 96% respectively. Therefore, only one new sterile line was formed, i.e. a radish cytoplasmic male sterile line Ronggan A105 of *Brassica oleracea,* where the sterile line can be propagated by pollination with Y3560.

### Embodiment 3:

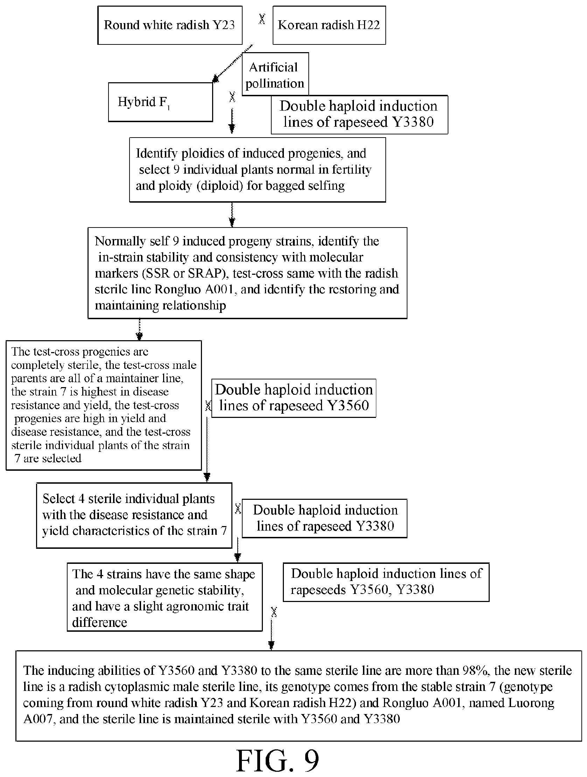

Referring to FIG. 1, FIG. 2, FIG. 4, FIG. 5 and FIG. 9, round white radish Y23 was crossed with Korean radish H22, the F₁ generation was artificially castrated at the flowering stage and pollinated with the double haploid induction line of rapeseed Y3380, the ploidies of induced progenies of the F₂ generation were identified with a flow cytometer at the seedling stage to find 10 individual plants, in which 1 individual plant was haploid, the remaining 9 were diploid, and were all in the shape of radish. The 9 individual plants were bagged for selfing at the flowering stage, the in-strain consistency and stability of the selfed progenies were identified to find that the 9 strains were highly uniform in in-strain consistency and stability, the pollen of the 9 strains was respectively test-crossed with a radish sterile line Rongluo A001, and the consistency and ploidy of the test-crossed progenies were identified to find that the 9 test-crossed progenies were all sterile, where the strain 7 had the highest disease resistance and yield. The test-crossed progenies of the strain 7 were continued to be pollinated and induced with the double haploid induction line of rapeseed Y3560 and bagged for isolation, and morphological separation was found in the induced progenies. 4 sterile individual plants with the characteristics of the strain 7 were selected and continued to be pollinated and induced with Y3380 to form 4 strains, and the in-strain consistency and stability of the 4 strains were identified to find that the strains were highly consistent and stable and were slightly different in agronomic traits, which mainly lied in leaf color and plant height. 5 individual plants were selected from each of the 4 stable strains, pollinated with Y3560 and Y3380 and bagged for isolation, seeds of 5 strains were collected in a mixed manner, the inducing effects of the inducing lines on the stable sterile lines were identified to find that the inducing efficiency of Y3560 was 94-99.8%, the inducing efficiency to one of the strains was 99.8% and this strain can be subjected to sterility maintenance with Y3560, the inducing efficiency of Y3380 is 93-99.7%, the inducing efficiency to one of the strains was 99.7% and this strain can be subjected to sterility maintenance with Y3380, it was finally found that the sterile lines of the two high inducing efficiencies were the same sterile line, the sterile line can be maintained sterile with Y3560 and Y3380, the new sterile line was a radish cytoplasmic male sterile line, its genotype came from the above bred stable strain 7 (the genotype of which came from round white radish Y23 and Korean radish H22) and Rongluo A001, named Luorong A007.

The breeding method of the rapeseed double inducing line in embodiments 2 and 3 was the same as that in Embodiment 1.

The method of the present invention can be used for obtaining cruciferous vegetable materials with application value in breeding or basic research rapidly (3 generations) with high efficiency and large scale; and the patent technology has a wide application range and is suitable for the entire cruciferous crops, including *Brassica oleracea,* cauliflower (broccoli), Chinese cabbage (celery cabbage, pakchoi), radish, mustard cruciferous vegetables (green vegetables, mustard, kohlrabi, Chinese kale, etc.), and the method is wide in application area and has a positive effect of promoting high yield and quality breeding of cruciferous vegetable crops.

The above embodiments further illustrate the above description of the present invention, but it should not be understood that the scope of the present invention is limited to the above embodiments. The techniques implemented based on the above all fall within the scope of the present invention.

## Claims

1. A method for breeding cruciferous vegetable materials and varieties with a double haploid induction line of rapeseed, comprising the following steps:
1) collecting breeding resources of cruciferous vegetables and identifying the characteristics of resource materials, classifying and numbering the resource materials having different sources and a great genetic difference of agronomic traits, investigating the flowering time, and retarding to sow the double haploid induction line of rapeseed according to the flowering time, the sowing time of rapeseed double haploids being generally October 20 to November 5 of the previous year, which can ensure flower synchronization with cruciferous vegetables in the next year;
2) artificially castrating the resource materials of cruciferous vegetables collected in step 1) at the initial flowering stage, directly pollinating sterile resources, performing bagging isolation for 2-4 days, then performing pollination with the double haploid induction line of rapeseed, and harvesting induced progenies from the bagged individual plants;
3) planting the induced progenies harvested in step 2), identifying the ploidies of the induced progenies with a flow cytometer at the seedling stage to eliminate polyploids, haploids and plants with rapeseed characteristics, and selecting individual plants normal in fertility and ploidy for bagged selfing or bud peeling forced selfing at the bud stage;
4) planting strains of normal selfing progenies of the induced progenies in step 3), identifying the in-strain stability and consistency, identifying the consistency in the strains with molecular markers, test-crossing the stable strains as male parents with a stable cytoplasmic male sterile line as female parents, and harvesting test-cross progeny seeds;
5) planting the test-cross progeny seeds in step 4), and identifying the fertility of the test-cross progeny plants, wherein if the test-cross progenies are completely sterile, the corresponding male parents in step 4) are a maintainer line of the sterile type, and meanwhile the selfing setting rate of the maintainer line is identified; wherein if the test-cross progenies are completely fertile, the corresponding male parents in step 4) are a restorer line of the sterile type, and the corresponding restorer line is eliminated; wherein if the test-cross progenies are sterile incompletely, the corresponding male parents in step 4) are not restored or maintained, and are eliminated or crossed to breed new maintainer lines;
6) test-matching the maintainer line bred in step 5) with the same type of sterile line to breed a new hybrid combination or variety with the characteristics of yield, quality and disease resistance;
7) test-crossing the maintainer line bred in step 5) with the same type of sterile line, and then according to the agronomic traits of the test-cross progenies, performing back crossing or multi-generation back crossing on the maintainer line with the test-cross progenies, or pollinating the sterile individual plants of test crossing, back crossing or multi-generation back crossing with the double haploid induction line of rapeseed at the test-cross first generation, and isolating same by bagging;
8) planting individual plant seeds harvested in step 7), identifying the fertility at the flowering stage, continuing to pollinate and induce sterile individual plants with the double haploid induction line of rapeseed, bagging the individual plants for isolation, and harvesting seeds from the individual plants;
9) identifying the in-strain stability and consistency of the second induced and sterile individual plant progenies in step 8) through agronomic traits and molecular markers, forming a new cytoplasmic male sterile line of cruciferous vegetables for in-strain stable sterile strains having consistent agronomic traits, and performing third induced pollination on the sterile strains with the double haploid induction line of rapeseed to identify the inducing efficiency;
10) identifying the inducing capability of the inducing line to the sterile strains for the progenies of third induction in step 9), and maintaining the genetic characteristics and infertility of the newly formed sterile strains (or stable sterile line) using the double haploid induction line of rapeseed if the agronomic traits and the infertility in the third induced progeny strains are highly consistent, and the inducing capability exceeds 98% (a ratio of plants with highly consistent agronomic traits and infertility to the total induced progenies);
11) pollinating the sterile strains formed after second induction in step 8) with the double haploid induction line of rapeseed to maintain the infertility thereof, the genetic characteristics of the sterile strains (or stable sterile line) being irrelevant to the double haploid induction line of rapeseed, the sterile strains having the traits of the maintainer line (temporary maintainer line) in step 7), and having a genetic difference from the maintainer line (temporary maintainer line), or containing 50-99% of nuclear genes of the maintainer line (temporary maintainer line), the content of the nuclear genes of the maintainer line (temporary maintainer line) depending on the number of back-crossing generations of the maintainer line (temporary maintainer line) and the sterile individual plants, and the sterile strains also containing nuclear genes of the stable cytoplasmic male sterile line in step 4) besides the nuclear genes of different degrees of the maintainer line (temporary maintainer line);
12) maintaining the stable cytoplasmic male sterile line with genetic differences formed in step 9) with the same maintainer line (double haploid induction line of rapeseed) according to the inducing capability (more than 98%) of the double haploid induction line of rapeseed to the cytoplasmic male sterile line, the double haploid induction line of rapeseed becoming a universal maintainer line of cytoplasmic male sterile lines; meanwhile, maintaining a plurality of genetically stable cytoplasmic male sterile lines with different genetic backgrounds with the same maintainer line;
13) breeding new cruciferous vegetable varieties with yield potential, disease resistance and stress resistance to realize two-line matching breeding of new hybrid varieties of cruciferous vegetables according to the material characteristics having different sources and a great genetic difference of agronomic traits in step 1) and the test matching of the corresponding source-induced stable maintainer line and multiple new stable cytoplasmic male sterile lines formed in step 12);
a method for breeding the above-mentioned double haploid induction line of rapeseed, comprising the following steps:
(1) breeding an early generation stable line with the parthenogenesis genetic characteristic:
a. artificially doubling chromosomes of hybrid F₁ generation seeds of two rapeseed parent materials on a medium by using a chromosome doubling inducer to obtain doubled F₁ generation plants;
b. selfing or forcedly selfing the doubled F₁ generation plants to obtain an F₂ generation, performing field planting observation on the F₂ generation, identifying the fertility of each individual plant, selecting fertile progenies and selfing same to obtain an F3 generation, identifying the homozygosity of the F3 generation by morphology, cytology and molecular markers, performing polymerase chain reaction amplification on progeny DNA, and observing the type and number of DNA bands of the individual plants under the amplification of each specific primer by electrophoresis, which shows that each individual plant is a hybrid progeny of two parents, and the molecular marker maps of the individual plants are consistent, indicating that these individual plants are of a homozygous line, i.e. an early generation stable line;
c. reciprocally crossing the obtained early generation stable line with at least 10 conventional homozygous stable lines of rapeseed, and identifying the genetic characteristics of the early generation stable line at the F₁ and F₂ generations, i.e., identifying whether there is the parthenogenesis characteristic, wherein if F₁ is separated and part of stable strains appear in the F₂ generation in the reciprocal crossing, the corresponding early generation stable line is an early generation stable line with the parthenogenesis genetic characteristic of parthenogenesis;
(2) breeding polyploid rapeseed with dominant genetic traits, parthenogenesis genetic characteristic and ploidy genetic stability:
a. crossing the early generation stable line with the parthenogenesis genetic characteristic with rapeseed with dominant traits to obtain hybrid F₁ generation seeds, and artificially doubling chromosomes of the hybrid F₁ seeds on a medium by using a chromosome doubling inducer to obtain doubled F₁ plants with dominant traits;
b. identifying the chromosome ploidies of the doubled F₁ plants with dominant traits through microscopic observation or a flow cytometer, selecting polyploid plants with dominant traits, and eliminating abnormal doubled plants, aneuploid plants and doubled plants without dominant traits, the polyploid plants with dominant traits being hexaploid or octoploid rapeseed plants with ploidy genetic stability, good setting property, parthenogenesis genetic characteristic and dominant traits;
(3) identifying the double haploid induction line of rapeseed and measuring the inducing capability:
a. the dominant traits in the polyploid plants with ploidy genetic stability, parthenogenesis genetic characteristic and dominant traits can be used for removing hybrid plants generated in the test-cross progenies, and if dominant plants or aneuploid plants appear in the test-cross progenies, it indicates that the plants are generated by the polyploid plants and female parents and are removed; and
b. if the individual plant test-cross progenies are completely sterile but have normal ploidies, i.e. diploid or tetraploid rapeseed, and do not have dominant traits, it indicates that the genes of the corresponding male parents of the test-cross progenies do not enter the test-cross progenies, wherein the dominant polyploid plants are of the double haploid induction line of rapeseed.

2. The method for breeding cruciferous vegetable materials and varieties with a double haploid induction line of rapeseed according to claim 1, wherein the double haploid induction line of rapeseed is bred by artificially doubling chromosomes of hybrid F₁ generation seeds of two parent materials, or hybrid F₁ generation seeds obtained by crossing the early generation stable line with the parthenogenesis genetic characteristic with rapeseed with dominant traits, on a medium by using a chromosome doubling inducer, and the specific method is as follows:
1) disinfecting the surfaces of the seeds with 75% alcohol for 25-40 seconds, disinfecting same with 0.1% mercury bichloride for 12-17 minutes, then washing away the mercury bichloride on the surfaces of the seeds with sterile water, sucking the water on the surfaces of the seeds with sterile paper, and then inoculating a first medium with the seeds;
2) allowing the seeds to root and sprout on the first medium under the culture conditions: temperature 23-25°C, daylight illumination 12-16 hours, light intensity 2000-3000 lux, night dark culture 8-12 hours, until the plants grow to 1-2 true leaves, and cutting the plants from the hypocotyls for continuing to grow on a second medium;
3) inserting the cut plants into the second medium to continue the culture, and after lateral buds are differentiated, transferring the lateral buds and the plants to a third medium for rooting culture; and
4) hardening seedlings of the plants at room temperature for 3-7 days after the plants grow thick roots after two weeks of rooting culture, taking the plants out, washing away the medium on the plants with tap water, soaking the plants in a soaking buffer solution for 15-30 minutes, and then transplanting the plants to a greenhouse, the greenhouse having a temperature of 16-25°C and a relative humidity of 60-80%, which can ensure that the survival rate of transplanting is 95% or above;
the first medium consists of the following components:
| | |
|---|---|
| MS medium | 1 L |
| 6-benzyl adenine | 0.5-1.5 mg |
| chromosome doubling inducer | 30-70 mg |
| sucrose | 20-30 g |
| agar | 8-10 g, |
the pH value of the first medium is 5.8-6.0;
the second medium consists of the following components:
| | |
|---|---|
| MS medium | 1 L |
| 6-benzyl adenine | 0.5-1 mg |
| chromosome doubling inducer | 20-40 mg |
| sucrose | 20-30 g |
| agar | 8-10 g, |
the pH value of the second medium is 5.8-6.0;
the third medium consists of the following components:
| | |
|---|---|
| MS medium | 1 L |
| α-naphthaleneacetic acid | 0.03-0.5 mg |
| chromosome doubling inducer | 5-20 mg |
| sucrose | 20-30 g |
| agar | 8-10 g, |
the pH value of the third medium is 5.8-6.0;
the soaking buffer solution consists of the following components:
| | |
|---|---|
| water | 1 L |
| famoxadone or curzate | 0.6-1.2 g |
| α-naphthaleneacetic acid | 0.5-1 mg. |

3. The method for breeding cruciferous vegetable materials and varieties with a double haploid induction line of rapeseed according to claim 1 or 2, wherein the chromosome doubling inducer is at least one of colchicine, trifluralin and oryzalin.
